# EUROPEAN PATENT SPECIFICATION

(11) **EP 4 268 869 B1**
(45) Date of publication and mention of the grant of the patent: **05.11.2025**
(21) Application number: 20966519.9
(22) Date of filing: 24.12.2020
(51) Int. Cl.: A61M 16/00, A61B 5/08, G16H 40/60, G16H 50/00

(54) **MEDICAL VENTILATION DEVICE CONFIGURED TO DISPLAY PATIENT-VENTILATOR ASYNCHRONY, AND DISPLAY METHOD THEREFOR**
MEDIZINISCHE BEATMUNGSVORRICHTUNG ZUR ANZEIGE EINER ASYNCHRONIE ZWISCHEN PATIENTEN UND BEATMUNGSGERÄT, UND ANZEIGEVERFAHREN DAFÜR
DISPOSITIF DE VENTILATION MÉDICALE CONÇU POUR AFFICHER UNE ASYNCHRONIE ENTRE LE PATIENT ET LE VENTILATEUR, ET PROCÉDÉ D'AFFICHAGE ASSOCIÉ

(43) Date of publication of application: 01.11.2023
(73) Proprietor: Shenzhen Mindray Bio-Medical Electronics Co., Ltd., Shenzhen, Guangdong 518057 (CN)
(72) Inventor: PAN, Ruiling, Shenzhen, Guangdong 518057 (CN); LI, Lan, Shenzhen, Guangdong 518057 (CN); LIU, Jinglei, Shenzhen, Guangdong 518057 (CN); HUANG, Zhiwen, Shenzhen, Guangdong 518057 (CN)
(74) Representative: KIPA AB
(86) International application number: PCT/CN2020/139150
(87) International publication number: WO 2022/133947

(56) References cited:
- WO-A1-2012/074958
- WO-A1-2017/068464
- WO-A1-2019/094736
- WO-A1-2020/037513
- CN-A- 103 169 476
- CN-A- 105 852 799
- CN-A- 109 718 440
- CN-A- 110 290 824
- US-A1- 2003 000 526
- US-A1- 2012 133 519
- US-A1- 2014 150 795
- US-A1- 2015 202 394
- US-A1- 2019 274 585

## Description

### TECHNICAL FIELD

The disclosure relates to medical devices, and more particularly to a display method for a ventilation device, and a ventilation device.

### BACKGROUND

Patient-ventilator asynchrony, also known as patient-machine asynchrony, refers to asynchronous phenomenon between ventilation provided by the ventilator and autonomous respiration of the patient, during a mechanical ventilation process for the patient. The occurrence of patient-ventilator asynchrony can affect a state of illness of the patient, cause discomfort, increase respiratory work, and prolong usage time of the ventilator. If the patient-ventilator asynchrony is severe, it can aggravate the lung injury in the patient and increase mortality. Therefore, it is very important to identify the phenomenon of patient-ventilator asynchrony and inform medical staff of this phenomenon when the patient uses the ventilator for respiratory support.

At present, ventilators, anesthesia machines, or other respiratory support devices on the market usually only provide the user with ventilation waveforms. For experienced user, the waveform can be used to determine whether there is patient-ventilator asynchrony, but this method is not intuitive enough and user-friendly.

US2014/0150795 provides methods and systems that determine whether a patient is asynchronous with a ventilator. In certain embodiments, a ventilation system may determine whether a double-triggering event has occurred between the patient and the ventilator.

WO2017068464 provides a mechanical ventilation device including a mechanical ventilator. At least one microprocessor is programmed to analyze the airway pressure and/or airway air flow to detect a spontaneous respiration anomaly. A display component is configured to display an indication of a spontaneous respiration anomaly detected by the anomaly detection component.

US20190274585 describes improved systems and methods for displaying respiratory data to a clinician in a ventilatory system. The disclosure describes novel systems and methods for determining and displaying ineffective patient inspiratory or expiratory efforts or missed breaths in a manner easily deciphered by a clinician.

WO2012/074958 describes systems and methods for monitoring and evaluating ventilatory parameters, analyzing those parameters and providing useful notifications and recommendations to clinicians. According to embodiments, a ventilator may be configured to monitor and evaluate diverse ventilatory parameters to detect double triggering and may issue notifications and recommendations suitable for a patient to the clinician when double triggering is implicated. The suitable notifications and recommendations may further be provided in a hierarchical format.

US2015/0202394 describes systems and methods for monitoring and evaluating ventilatory parameters, analyzing those parameters and providing useful notifications and recommendations to clinicians. According to embodiments, a ventilator may be configured to monitor and evaluate diverse ventilatory parameters to detect asynchrony and may issue notifications and recommendations suitable for a patient to the clinician when asynchrony is implicated.

WO2019094736 describes a system that automatically detects patient-ventilator asynchrony and trends in patient-ventilator asynchrony. The present disclosure describes a framework that uses pressure, flow, and volume waveforms to detect patient-ventilator asynchrony and the presence of secretions in the ventilator circuit.

### SUMMARY OF THE INVENTION

The present invention provides a ventilation device as defined in claim 1 and a display method for a ventilation device as defined in claim 11. Further preferred embodiments of the present invention are defined in the dependent claims.

### SUMMARY OF THE DISCLOSURE

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

According to a first aspect, an embodiment provides a display method for a ventilation device, including:
acquiring a parameter of a patient which characterizes occurrence of patient-ventilator asynchrony during a ventilation process, wherein the parameter includes at least one of: a ventilation parameter of the ventilation device and a physiological parameter of the patient;
determining whether the patient-ventilator asynchrony occurs according to the parameter;
outputting, when the patient-ventilator asynchrony occurs, indication information, which is associated with the patient-ventilator asynchrony, and parameter waveforms, which are generated according to the parameter;
wherein outputting indication information, which is associated with the patient-ventilator asynchrony, and parameter waveforms, which are generated according to the parameter, comprises: displaying, on a display interface, the indication information, which is associated with the patient-ventilator asynchrony, and the parameter waveforms of a period of time; wherein the patient-ventilator asynchrony occurs within said period of time;
wherein displaying, on a display interface, the indication information, which is associated with the patient-ventilator asynchrony, and the parameter waveforms of a period of time, comprises:
   recording a time point when the patient-ventilator asynchrony occurs; and displaying a first mark at a position of the parameter waveforms, which position corresponds to the time point when the patient-ventilator asynchrony occurs;
   wherein displaying a first mark at a position of the parameter waveforms, which position corresponds to the time point when the patient-ventilator asynchrony occurs, comprises: recognizing a type of the patient-ventilator asynchrony occurred; and displaying the first mark, which corresponds to the type of the patient-ventilator asynchrony occurred, which type is recognized according to a first correspondence relationship, wherein the first correspondence relationship characterizes a first mark which corresponds to different types of patient-ventilator asynchrony; wherein said first mark, which corresponds to different types of patient-ventilator asynchrony, differs in at least one characteristic of color, pattern, and brightness.

According to a second aspect, an embodiment provides a ventilation device, including:
a gas source interface, which is connected with an external gas source;
a patient interface, which is connected with a respiratory system of a patient;
a respiratory line, which is connected with both the gas source interface and the patient interface, so as to input a gas from the external gas source to the patient and receive a gas exhaled by the patient;
a drive apparatus, which is configured to provide respiratory support power, so as to control to input the gas from the external gas source to the patient, recycle the gas exhaled by the patient or output the gas exhaled by the patient to external environment;
a parameter acquisition device, which is configured to acquire a parameter of the patient which characterizes occurrence of patient-ventilator asynchrony during a ventilation process, wherein the parameter includes at least one of: a ventilation parameter of the ventilation device and a physiological parameter of the patient;
a processor, which is configured to determine whether the patient-ventilator asynchrony occurs according to the parameter, and output indication information, which is associated with the patient-ventilator asynchrony, and parameter waveforms, which are generated according to the parameter, when the patient-ventilator asynchrony occurs;
wherein outputting indication information, which is associated with the patient-ventilator asynchrony, and parameter waveforms, which are generated according to the parameter, comprises: displaying, on a display interface, the indication information, which is associated with the patient-ventilator asynchrony, and the parameter waveforms of a period of time; wherein the patient-ventilator asynchrony occurs within said period of time;
wherein displaying, on a display interface, the indication information, which is associated with the patient-ventilator asynchrony, and the parameter waveforms of a period of time, comprises: recording, through a memory of the ventilation device, a time point when the patient-ventilator asynchrony occurs; and displaying a first mark at a position of the parameter waveforms, which position corresponds to the time point when the patient-ventilator asynchrony occurs;
wherein displaying a first mark at a position of the parameter waveforms, which position corresponds to the time point when the patient-ventilator asynchrony occurs, comprises: recognizing, through the processor, a type of the patient-ventilator asynchrony occurred; and displaying, on the display interface, the first mark, which corresponds to the type of the patient-ventilator asynchrony occurred, which type is recognized through the processor according to a first correspondence relationship, wherein the first correspondence relationship characterizes a first mark which corresponds to different types of patient-ventilator asynchrony, wherein the first correspondence relationship is pre-stored in the memory; wherein said first mark, which corresponds to different types of patient-ventilator asynchrony, differs in at least one characteristic of color, pattern, and brightness.

In the above embodiment, if the patient-ventilator asynchrony occurs, indication information which is associated with the patient-ventilator asynchrony and parameter waveforms which are generated according to the parameter when the patient-ventilator asynchrony occurs are outputted; wherein the parameter includes at least one of: a ventilation parameter of the ventilation device and a physiological parameter of the patient. This is greatly helpful for subsequent adjustment of ventilation parameter or evaluation of patient condition.

### BRIEF DESCRIPTION OF THE DRAWINGS

FIG. 1 is a schematic diagram of a ventilation device in an embodiment.
FIG. 2 is a schematic diagram of a parameter acquisition device in an embodiment.
FIG. 3 shows a display interface for presenting a time point when patient-ventilator asynchrony occurs in an embodiment.
FIG. 4 shows a display interface for presenting a time point when patient-ventilator asynchrony occurs in another embodiment.
FIG. 5 shows a display interface for presenting a time point when patient-ventilator asynchrony occurs in another embodiment.
FIG. 6 shows a display interface for presenting a time point when patient-ventilator asynchrony occurs in another embodiment.
FIG. 7 shows a display interface for presenting a time point when patient-ventilator asynchrony occurs in another embodiment.
FIG. 8 shows a display interface for presenting a frequency for the occurrence of patient-ventilator asynchrony in an embodiment.
FIG. 9 shows a display interface for presenting information for a degree of patient-ventilator asynchrony in an embodiment.
FIG. 10 shows a display interface for presenting information for a degree of patient-ventilator asynchrony in another embodiment.
FIG. 11 shows a display interface for analyzing patient-ventilator asynchrony in an embodiment.
FIG. 12 shows a display interface after combining indication information in an embodiment.
FIG. 13 shows a display interface after combining indication information in another embodiment.
FIG. 14 shows a display interface after combining indication information in another embodiment.
FIG. 15 shows a display interface after combining indication information in another embodiment.
FIG. 16 is a flowchart of a display method for a ventilation device in an embodiment.
   1. First mark;
   2. Second mark;
   3. Third mark;
   10. Gas source interface;
   20. Drive device;
   30. Respiratory line; 30a, inspiratory branch; 30b, expiratory branch; 31. Carbon dioxide absorber; 32. Check valve;
   40. Patient interface;
   50. Parameter acquisition device;
   60. Memory;
   70. Processor.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, so as to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the characteristics, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are used to distinguish different objects, rather than to describe a specific order. In addition, the terms "connection", and "linkage" mentioned in this application include direct and indirect connection (linkage), unless otherwise specified.

The abbreviation for the patient-ventilator asynchrony referred to in this disclosure is AI. When referring to various types of patient-ventilator asynchrony, the abbreviation for invalid triggering is IE, the abbreviation for double triggering is DT, the abbreviation for low flow rate is IF, the abbreviation for abnormal triggering is AT, and the abbreviation for reverse triggering is RT.

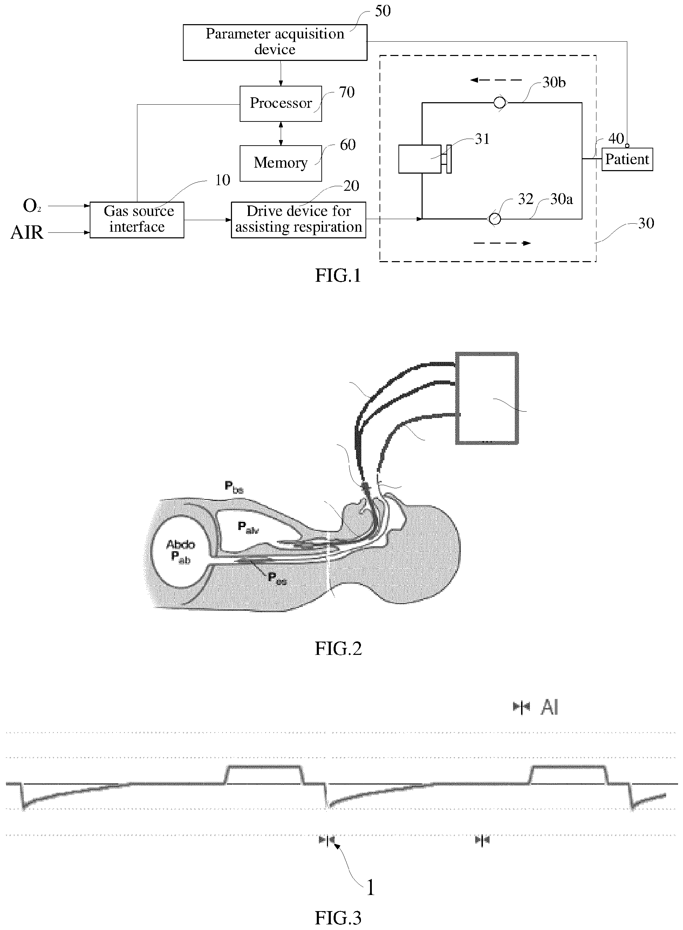

Please refer to the embodiment shown in FIG. 1, which provides a ventilation device (such as a ventilator, anesthesia machine, etc.), which includes a gas source interface 10, a drive device 20, a respiratory line 30, a patient interface 40, a parameter acquisition device 50, a memory 60, and a processor 70.

The gas source interface 10 is configured to connect to an external gas source (not shown in the figure), which provides a gas. This gas can usually be oxygen, air, etc. In some embodiments, the gas source uses a compressed gas cylinder or a central gas supply source to supply the gas to the ventilation device through the gas source interface 10. The types of gas supplied include oxygen (O2) and air, etc. The gas source interface 10 can include conventional components, such as a pressure gauge, pressure regulator, flow meter, pressure reducing valve, and proportional control protection device, which are respectively used to control flows of various gases (such as oxygen and air). The gas inputted from the gas source interface 10 enters the respiratory line 30 and forms a mixed gas with the original gas in the respiratory line 30.

The drive device 20 is configured to provide power for non-spontaneous respiration of the patient, maintain airway opened, that is, to drive the gas inputted from the gas source interface 10 and the mixed gas in the respiratory line 30 to the respiratory system of the patient, and to guide the exhaled gas to the respiratory line 30, thereby improving ventilation and oxygenation, preventing the patient from hypoxia and carbon dioxide accumulation. In a specific embodiment, the drive device 20 typically includes a machine-controlled ventilation module, and a gas flow pipeline of the machine-controlled ventilation module is connected with the respiratory line 30. During the surgery, when the patient has not regained autonomous respiration, the machine-controlled ventilation module is configured to provide respiration power for the patient. In some embodiments, the drive device 20 also includes a manual ventilation module, and a gas flow pipeline of the manual ventilation module is connected to the respiratory line 30. During the induction phase before intubation in the surgery process, it is usually necessary to use a manual ventilation module to assist the patient in respiration. When the drive device 20 includes both a machine-controlled ventilation module and a manual ventilation module, the machine-controlled or manual ventilation mode can be switched through a machine-controlled or manual switch (such as a three-way valve) to connect the machine-controlled ventilation module or manual ventilation module with the respiratory line 30, thereby controlling the respiration of the patient.

The respiratory line 30 includes an inspiratory branch 30a, an expiratory branch 30b, and a carbon dioxide absorber 31. The inspiratory branch 30a and the expiratory branch 30b are connected to form a closed loop, and the carbon dioxide absorber 31 is arranged inside a pipeline of the expiratory branch 30b. The mixed gas of fresh air introduced by the gas source interface 10 is inputted from an inlet of the inspiratory branch 30a and provided to the patient through the patient interface 40, which is arranged at an outlet of the inspiratory branch 30a. The patient interface 40 may be a mask, a nasal intubation tube or a tracheal intubation tube. In a preferred embodiment, a check valve 32 is arranged inside the inspiratory branch 30a, and opened during the inhalation phase and closed during the exhalation phase. The expiratory branch 30b is also arranged with the check valve 32, which is closed during the inhalation phase and opened during the exhalation phase. The inlet of the expiratory branch 30b is connected with the patient interface 40. When the patient exhales, the exhaled gas enters the carbon dioxide absorber 31 through the expiratory branch 30b, and carbon dioxide in the exhaled gas is filtered out by substances inside the carbon dioxide absorber 31. The filtered carbon dioxide gas is then recycled in the inspiratory branch 30a.

The parameter acquisition device 50 is configured to acquire a parameter used by the patient to characterize occurrence of patient-ventilator asynchrony during the ventilation process, including at least one of: a ventilation parameter of the ventilation device and a physiological parameter of the patient. The parameter can include but is not limited to at least one of (airway) flow rate, (airway) pressure, (pulmonary) volume, esophageal pressure, intragastric pressure, transpulmonary pressure, blood oxygen, respiratory rate, transdiaphragmatic pressure, and diaphragmatic electromyography. According to the different parameters that need to be acquired, different parameter acquisition devices 50 can be used. In some embodiments, the parameter acquisition device 50 includes a sensor interface 51 and a sensor for acquiring various respiratory information of patient, such as airway pressure and esophageal pressure. Specifically, the sensor interface 51 is respectively connected with a signal output terminal of a first pressure sensor 52a and a signal output terminal of a second pressure sensor 52d. As shown in FIG. 2, a first sampling tube 52b is inserted into trachea through oral cavity, and the first pressure sensor 52a is arranged inside the first sampling tube 52b to monitor a pressure inside the trachea (i.e., airway pressure), which equivalent to an alveolar pressure. Then the first pressure sensor 52a converts the acquired airway pressure to an electrical signal, and transmit the electrical signal to the sensor interface 51 through a first wire 52c. A second sampling tube 52e is inserted into esophagus through nasal cavity, and the second pressure sensor 52d is arranged inside the second sampling tube 52e to monitor a pressure inside the esophagus (i.e., esophageal pressure), which is equivalent to an intrathoracic pressure. The second pressure sensor 52d then converts the acquired esophageal pressure into an electrical signal and transmits it to the sensor interface 51 through the second wire 52f. In one embodiment, the sensor interface 51 may simply serve as a connector between the output terminals of the sensors and subsequent circuits (such as processor 70) without processing the signal. The sensor interface 51 can also be integrated into the processor 70 as an interface for accessing signals. In another embodiment, the sensor interface 51 may include an amplification circuit, a filtering circuit, and an A/D conversion circuit for amplifying, filtering, and analog-to-digital conversion processing of inputted analog signals, respectively. Of course, technicians should understand that the connection relationship between the amplification circuit, filtering circuit, and A/D conversion circuit can vary depending on specific designs of the circuits, or a certain circuit can be reduced. For example, the amplification circuit or filtering circuit can be reduced, thereby reducing its corresponding function. In addition, the sensor interface 51 can also access information which is acquired by other sensors, such as flow information and/or pressure information of the respiratory line 30. As specific products, the first pressure sensor 52a, the first sampling tube 52b, the second pressure sensor 52d, and the second sampling tube 52e can be parts of the ventilation device or external accessories independent of the ventilation device.

Memory 60 can be a tangible and non-transient computer-readable medium, such as a flash memory, RAM, ROM, EEPROM, etc.

The processor 70 is configured to determine whether patient-ventilator asynchrony occurs according to the parameter, and output indication information, which is associated with the patient-ventilator asynchrony, and parameter waveforms, which are generated according to the parameter when the patient-ventilator asynchrony occurs. For example, the processor 70 can be in signal connection with an external display device, so as to output the indication information and parameter waveforms to the display device. The types of patient-ventilator asynchrony mentioned above include but are not limited to invalid triggering, double triggering, abnormal triggering, reverse triggering, and low flow rate.

At present, various algorithms have been developed to determine whether corresponding patient-ventilator asynchrony occurs according to the aforementioned ventilation and/or physiological parameters. For example, invalid triggering, abnormal triggering, and reverse triggering of patient-ventilator asynchrony can be determined according to the airway pressure and airway flow rate, which is not further elaborated here.

In the present disclosure, the parameter waveforms, and the indication information which is associated with patient-ventilator asynchrony, are outputted simultaneously. In some embodiments, "outputting simultaneously" means simultaneously displaying, on a display interface, the indication information, which is associated with the patient-ventilator asynchrony, and the parameter waveforms of a period of time, wherein a time point, when the patient-ventilator asynchrony occurs, is within the period of time. That is to say, on the display interface, it is possible to see the abnormal waveforms displayed during the patient-ventilator asynchrony, as well as the indication information which is associated with the patient-ventilator asynchrony. This allows the user to associate the patient-ventilator asynchrony with indication information in mind. This indication information can provide the user with different information about the patient-ventilator asynchrony, as explained in detail below.

In some embodiments, the indication information can present a time point for the occurrence of the patient-ventilator asynchrony, and specifically includes follows.

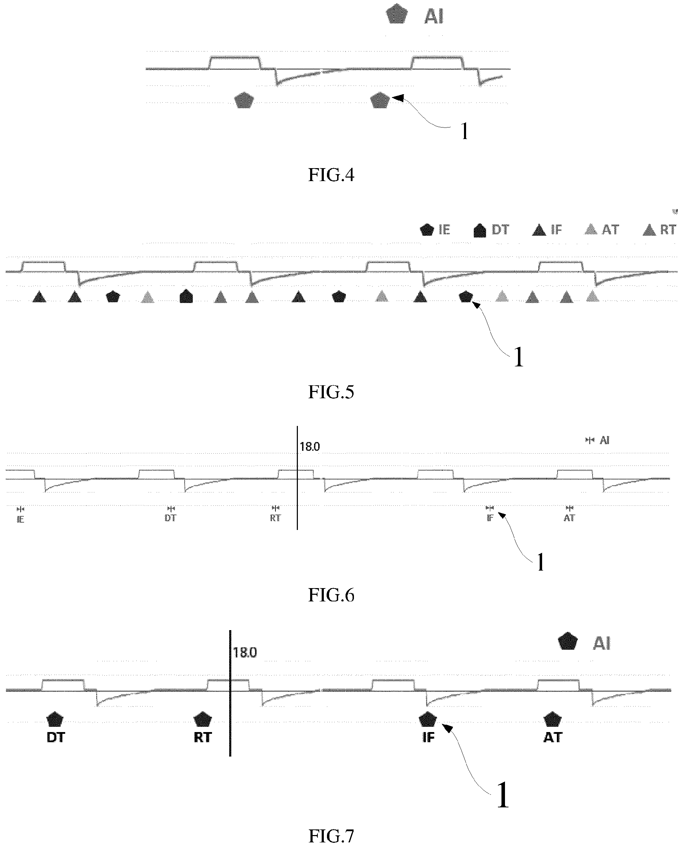

The processor 70 records a time point when the patient-ventilator asynchrony occurs, and then acquires positions of the parameter waveforms on the display interface, at each time point on a coordinate axis of time. Finally, the processor 70 displays a first mark 1 at a position of the coordinate axis, which position corresponds to the time point when the patient-ventilator asynchrony occurs. Wherein, the first mark 1 includes but is not limited to a word, number, symbol and animation. Take the display interface shown in FIG. 3 and FIG. 4 for example. The display interface shows real-time waveforms of the patient, and corresponding symbols present at corresponding positions of the coordinate axis, indicating that patient-ventilator asynchrony occurs at that time. Wherein, the first mark 1 in FIG. 3 has a symmetry axis and two triangles which are symmetrical about the symmetry axis, while the first mark 1 in FIG. 4 is a regular pentagon. In addition, the memory 60 can pre-store a first correspondence relationship, which represents the first mark 1 which corresponds to different types of the patient-ventilator asynchrony. The first marks 1 which correspond to the different types of the patient-ventilator asynchrony, differ in at least one characteristic of color, pattern, and brightness. After a patient-ventilator asynchrony occurs, the processor 70 recognizes the type of the patient-ventilator asynchrony occurred, and then displays the first mark 1 which corresponds to the type of the patient-ventilator asynchrony recognized according to first correspondence relationship. For example, the first marks 1 for different types of the patient-ventilator asynchrony can have the same shape but different colors. For example, as shown in FIG. 5, the first marks 1 for different types of patient-ventilator asynchrony have different shapes and colors. In other embodiments, there are many ways to distinguish each type of patient-ventilator asynchrony from the first marks 1, which are not elaborated here. In addition to real-time waveforms, it is also possible to display waveforms, such as frozen waveforms or historical flow rate waveforms on the display interface. For example, the frozen waveforms are shown in FIG.6 and FIG.7, in which the first mark 1 is displayed at the position of the coordinate axis of the waveform, which position corresponds to the time point when the patient-ventilator asynchrony occurs.

In some embodiments, this indication information can present a frequency for the occurrence of patient-ventilator asynchrony, including follows.

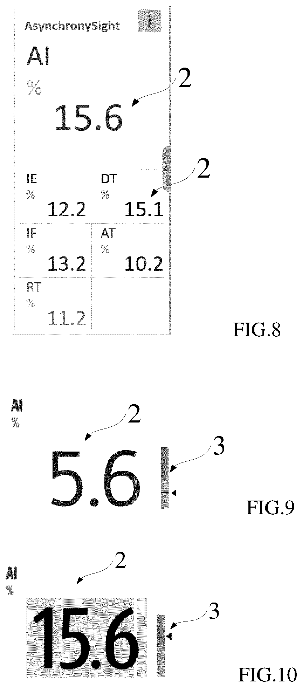

The processor 70 records a time point when the patient-ventilator asynchrony occurs, and then calculates a proportion of a number of respiratory circulation cycle(s) in a preset time period, in which cycle(s) the patient-ventilator asynchrony occurs, to a total number of respiratory circulation cycle(s) in the preset time period before the patient-ventilator asynchrony occurs, and obtains the frequency for the occurrence of the patient-ventilator asynchrony during the preset time period. For example, if the preset time period contains ten respiratory circulation cycles, and the patient-ventilator asynchrony occurs within six respiratory circulation cycles, the frequency for the occurrence of the patient-ventilator asynchrony is 60%. After the frequency for the occurrence of patient-ventilator asynchrony in the preset time period is acquired, a second mark 2, which qualitatively and/or quantitatively characterizes the frequency for the occurrence, is displayed on the display interface. The second mark 2 can be at least one of: dynamic trend graph, broken line graph, scatter graph, spider web graph, number, text, scale graph and dial graph, or can also be other ways that can qualitatively or quantitatively characterize the frequency for the occurrence of the patient-ventilator asynchrony. As shown in FIG. 8, it is a quantitative display of the frequency for the occurrence of patient-ventilator asynchrony. In this figure, the frequency for the occurrence of patient-ventilator asynchrony is directly displayed in a digital form, which is clear to user at a glance.

The frequency for the occurrence of the patient-ventilator asynchrony mentioned above is calculated by integrating different types of the patient-ventilator asynchrony, and can also be referred to as the total frequency for the occurrence of patient-ventilator asynchrony. For different types of patient-ventilator asynchrony, the frequency for the occurrence of each type of patient-ventilator asynchrony can also be calculated separately. Specifically, the memory 60 can pre-store a second correspondence relationship, which represents second marks 2 which correspond to each type of patient-ventilator asynchrony. It is easy to understand that different types of patient-ventilator asynchrony correspond to different second marks 2. The processor 70 recognizes the types of patient-ventilator asynchrony occurred within a preset time period, calculates a proportion of a number of respiratory circulation cycle(s), in which each type of patient-ventilator asynchrony occurs, to a total number of respiratory circulation cycles in a preset time period, and obtains the frequency for the occurrence of each type of patient-ventilator asynchrony during the preset time period. For example, the preset time period includes ten respiratory circulation cycles. If the patient-ventilator asynchrony occurs within six respiratory circulation cycles, the total frequency for the occurrence of patient-ventilator asynchrony is 60%. Among the six respiratory circulation cycles in which the patient-ventilator asynchrony occurs, four respiratory circulation cycles only have invalid triggering, and each respiratory circulation cycle in the other two respiratory circulation cycles has both abnormal triggering and double triggering. The frequency for the occurrence of invalid triggering is 40%, and the occurrence frequencies of abnormal triggering and double triggering are both 20%. After obtaining the frequency for the occurrence of different types of the patient-ventilator asynchrony within the preset time period, processor 70 displays the second mark 2 which corresponds to the type of the patient-ventilator asynchrony recognized according to the second correspondence relationship. As shown in FIG. 8, in an embodiment, the total frequency for the occurrence of the patient-ventilator asynchrony and the occurrence frequencies of different types of the patient-ventilator asynchrony are displayed on the same display interface. It can be seen that the total frequency for the occurrence of patient-ventilator asynchrony is 15.6%. In FIG. 8, the second mark 2 for each type of patient-ventilator asynchrony includes corresponding characters, where the characters of the second mark 2 corresponding to the invalid triggering are IE, and the characters of the second mark 2 corresponding to the double triggering are DT. The second marks 2 for other types of patient-ventilator asynchrony have the same principles.

Through the above methods, the user can clearly know the total frequency for the occurrence of the patient-ventilator asynchrony and the occurrence frequencies of different types of the patient-ventilator asynchrony.

In some embodiments, after obtaining the frequency for the occurrence of different types of the patient-ventilator asynchrony, a patient-ventilator asynchrony index is also introduced as indication information to characterize a degree of severity of the patient-ventilator asynchrony. In some embodiments, a sum of the occurrence frequencies of different types of the patient-ventilator asynchrony obtained above is used as the patient-ventilator asynchrony index. Obviously, the higher the patient-ventilator asynchrony index, the relatively higher the degree of severity of the patient-ventilator asynchrony.

In order to make the reminder of the degree of severity of the patient-ventilator asynchrony more obvious, in some embodiments, a third mark 3 is also displayed on the display interface, which includes but is not limited to text, number, symbol, and animation. The processor 70 can determine whether the patient-ventilator asynchrony index exceeds a preset threshold. If yes, a characteristic of the third marker 3 can be changed. The characteristic of the third marker 3 can include but is not limited to a color characteristic, brightness characteristic, and pattern characteristic. For example, as shown in FIGS. 9 and 10, the third marker 3 includes a rectangular box and a pointer. An upper half of the rectangular box is of one color (such as red), and a lower half of the rectangular box is of another color (or can also be transparent). Depending on the patient-ventilator asynchrony index, the pointer stays at different heights of the rectangular box. As shown in FIG. 9, when the patient-ventilator asynchrony index does not reach the preset threshold, the display position of the pointer is in the lower half of the rectangular box, as shown in FIG. 10. When the patient-ventilator asynchrony index exceeds the preset threshold, the display position of the pointer is in the upper half of the rectangular box. In this way, the user does not need to see the frequency for the occurrence of patient-ventilator asynchrony or the specific figure of the patient-ventilator asynchrony index, but can more intuitively obtain the degree of severity of patient-ventilator asynchrony through the characteristic change of the third marker 3.

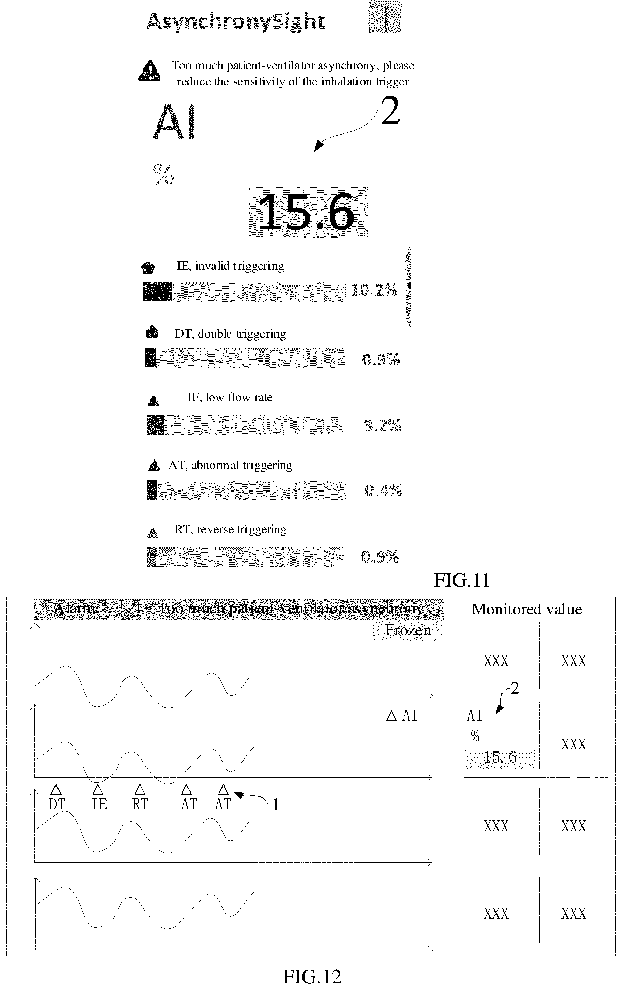

In some embodiments, after obtaining the frequency for the occurrence of different types of the patient-ventilator asynchrony, indication information is also used to analyze and compare patient-ventilator asynchrony event(s). Specifically, the processor 70 can rank different types of the patient-ventilator asynchrony according to their occurrence frequencies, and display the ranking results on the display interface. The user can take corresponding measures according to the ranking results, as shown in FIG. 11, the frequency for the occurrence of each type of patient-ventilator asynchrony decreases from top to bottom. The user can preferentially take measures to reduce the types of patient-ventilator asynchrony that occur most frequently. In some embodiments, the memory 60 can pre-store a third correspondence relationship, which characterizes adjustment suggestions for ventilation parameters corresponding to various ranking results. After obtaining ranking results of different types of the patient-ventilator asynchrony, the processor 70 can display the adjustment suggestions for ventilation parameters according to the third correspondence relationship, enabling the user, who do not need to have too deep medical knowledge, to use the above-mentioned ventilation device well. The usability of the ventilation device in this embodiment is improved. The ventilation parameters recommended can be displayed in the form of text, animation, or image. For example, in FIG. 11, the text "Too much patient-ventilator asynchrony, please reduce the sensitivity of the inhalation trigger" is used as the ventilation parameters recommended. Furthermore, the corresponding relationships between various ranking results and adjustment suggestions for ventilation parameters can include but are not limited to the following. (1) Provide adjustment suggestions for ventilation parameters according to the type of patient-ventilator asynchrony ranked first. (2) Provide adjustment suggestions for ventilation parameters according to the types of patient-ventilator asynchrony, ranked second and third, based on a certain type of patient-ventilator asynchrony ranked first. (3) Provide a corresponding adjustment suggestion for ventilation parameters for each possible ranking result. In other embodiments, a pre-generated ventilation parameter adjustment interface can also be displayed according to an input instruction, which can instruct the user to make parameter adjustments. In some embodiments, the third correspondence relationship can also be prepared according to the physiological parameters of the patient, such as blood oxygen, pulse, and respiratory rate, to provide relevant recommendation for ventilation parameters.

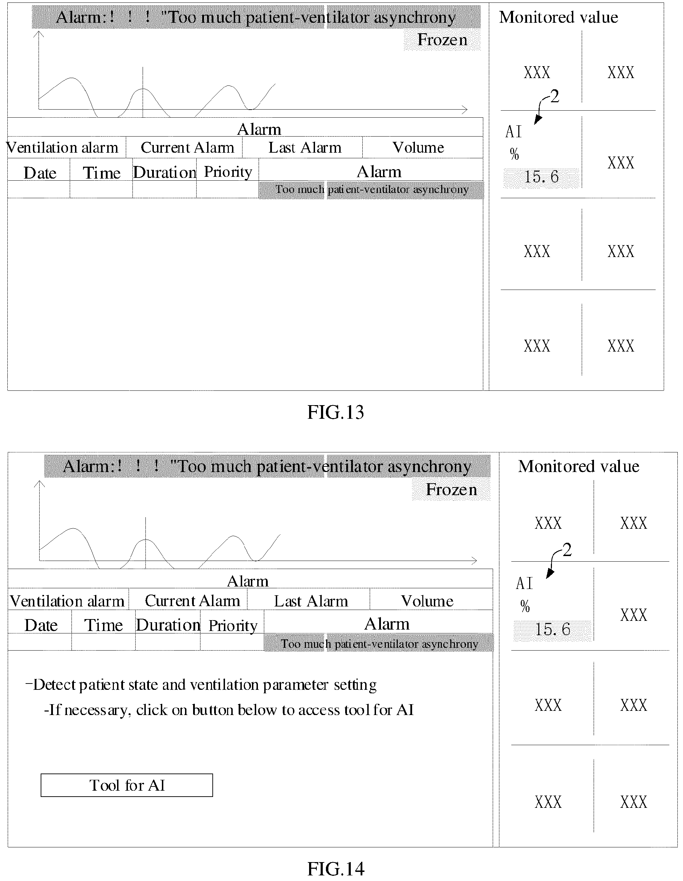

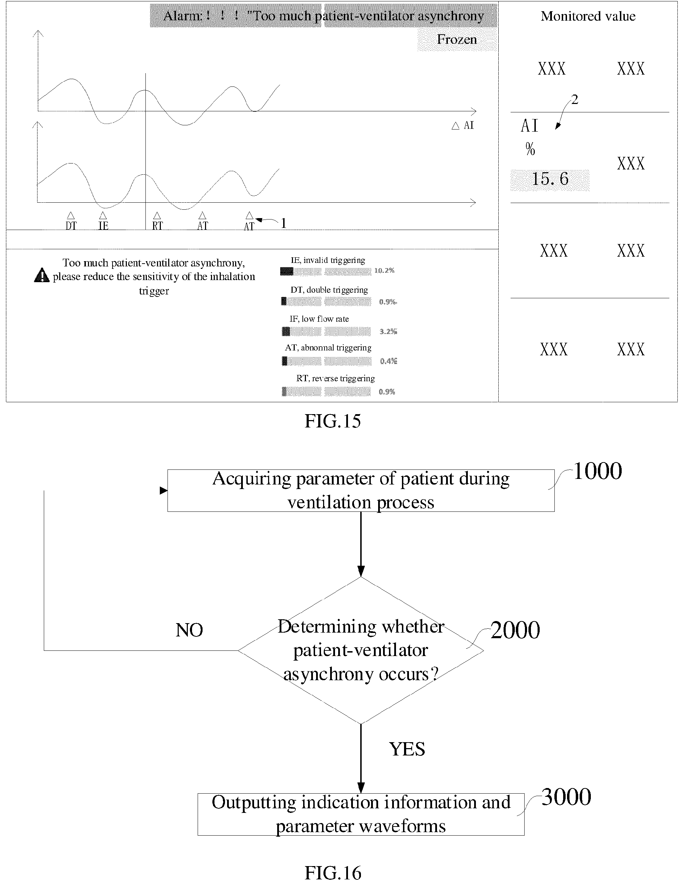

The indication information in the above embodiments can be displayed separately, and in some embodiments, the indication information in the above embodiments can also be combined to provide the user with comprehensive indication information about patient-ventilator asynchrony, as shown in FIGS. 12 to 15. As shown in FIG. 12, the left area is frozen waveforms of parameter, while the right area is configured to display occurrence frequencies of different types of the patient-ventilator asynchrony and total frequency for the occurrence of patient-ventilator asynchrony. The first mark 1 of a triangle is displayed on a coordinate axis of a parameter waveform in the figure. In this interface, the total frequency for the occurrence of patient-ventilator asynchrony calculated is 15.6%, which reaches the degree of severity, and an alarm is required in this embodiment. Therefore, alarm information "!!! Excessive patient-ventilator asynchrony" is displayed at a top of the display interface. That is to say, FIG. 12 shows the time point for the occurrence, frequency for the occurrence, and related alarm information of patient-ventilator asynchrony. According to this, the display interface in FIG. 12 also provides the user with corresponding jump or pop-up buttons. For example, the user can click on the area where the total frequency for the occurrence of patient-ventilator asynchrony occurs on the display interface, and a tool for analyzing patient-ventilator asynchrony pops up, as shown in FIG. 15. In this display interface, the tool for analyzing patient-ventilator asynchrony is displayed, and adjustment suggestions for ventilation parameters are displayed. For example, the user can click on the area where the alarm information is located on the display interface to jump to a detailed alarm information interface shown in FIG. 13, which displays more detailed alarm information. If the user further clicks on the alarm information in the detailed alarm information interface, it jumps to the display interface shown in FIG. 14, which displays general adjustment suggestions for ventilation parameter, and the display interface also provides an entry point for the tool for analyzing patient-ventilator asynchrony. After clicking on this button, the tool for analyzing patient-ventilator asynchrony as shown in FIG. 15 pops up.

The above is an example of a combination of indication information. In the above combination, each display interface can be displayed in separate screens, or only a portion of the display interface can be displayed on the same screen at a time. The present disclosure is not limited to the combination method mentioned above, and the above indication information can also be output in the form of sound or light.

The present disclosure also provides a display method for a ventilation device, as shown in FIG. 16, including following step.

In step 1000, a parameter of a patient which characterizes occurrence of patient-ventilator asynchrony during a ventilation process, is acquired. The parameter includes at least one of: a ventilation parameter of the ventilation device and a physiological parameter of the patient. The parameter can include but are not limited to at least one of (airway) flow rate, (airway) pressure, (pulmonary) volume, esophageal pressure, intragastric pressure, transpulmonary pressure, blood oxygen, respiratory rate, transdiaphragmatic pressure, and diaphragmatic electromyography.

Step 2000, whether the patient-ventilator asynchrony occurs, is determined according to the parameter. If the patient-ventilator asynchrony occurs, execute step 3000. If patient-ventilator asynchrony does not occur, execute step 1000.

At present, various algorithms have been developed to determine whether corresponding patient-ventilator asynchrony occurs according to the aforementioned ventilation and/or physiological parameters. For example, types of patient-ventilator asynchrony, such as invalid triggering, abnormal triggering, and reverse triggering, can be determined according to airway pressure and flow rate, which is not further elaborated here.

Step 3000, indication information, which is associated with the patient-ventilator asynchrony and parameter waveforms which are generated according to the parameter, when the patient-ventilator asynchrony occurs, are outputted. In step 3000, the parameter waveforms, and the indication information which is associated with patient-ventilator asynchrony, are outputted simultaneously. In some embodiments, "outputting simultaneously" means simultaneously displaying, on a display interface, the indication information, which is associated with the patient-ventilator asynchrony, and the parameter waveforms of a period of time, wherein a time point, when the patient-ventilator asynchrony occurs, is within the period of time. That is to say, on the display interface, it is possible to see the abnormal waveforms displayed during the patient-ventilator asynchrony, as well as the indication information which is associated with the patient-ventilator asynchrony. This allows the user to associate the patient-ventilator asynchrony with indication information in mind. This indication information can provide the user with different information about the patient-ventilator asynchrony, as explained in detail below.

In some embodiments, step 3000 includes following steps:
step 3100, recording a time point when the patient-ventilator asynchrony occurs;
step 3110, acquiring a position of the parameter waveforms on the display interface, at each time point on a coordinate axis of time;
step 3120, displaying a first mark at a position of the coordinate axis, which position corresponds to the time point when the patient-ventilator asynchrony occurs.

Wherein, the first mark 1 includes but is not limited to word, number, symbol and animation. Take the display interface shown in FIG. 3 and FIG. 4 for example. The display interface shows real-time waveforms of the patient, and corresponding symbols present at corresponding positions of the coordinate axis, indicating that patient-ventilator asynchrony occurs at that time. Wherein, the first mark 1 in FIG. 3 has a symmetry axis and two triangles which are symmetrical about the symmetry axis, while the first mark 1 in FIG. 4 is a regular pentagon. In addition, a first correspondence relationship, which represents the first mark 1 which corresponds to different types of the patient-ventilator asynchrony, can be pre-stored. The first mark 1 which corresponds to the different types of the patient-ventilator asynchrony, differs in at least one characteristic of color, pattern, and brightness. After the occurrence of patient-ventilator asynchrony, the type of patient-ventilator asynchrony occurred, can be recognized, and then the first mark 1 which corresponds to the type of the patient-ventilator asynchrony recognized according to first correspondence relationship can be displayed. For example, the first mark 1 for different types of patient-ventilator asynchrony can have the same shape but different colors. For example, as shown in FIG. 5, the first mark 1 for different types of patient-ventilator asynchrony has different shapes and colors. In other embodiments, there are many ways to distinguish each type of patient-ventilator asynchrony from the first mark 1, which is not elaborated here. In addition to real-time waveforms, it is also possible to display waveforms, such as frozen waveforms or historical flow rate waveforms on the display interface. For example, the frozen waveforms are shown in FIG.6 and FIG.7, in which the first mark 1 is displayed at the position of the coordinate axis of the waveform, which position corresponds to the time point when the patient-ventilator asynchrony occurs.

In some embodiments, step 3000 includes:
step 3200, recording a time point when the patient-ventilator asynchrony occurs;
Step 3210, calculating a proportion of a number of respiratory circulation cycle(s) in a preset time period, in which cycle(s) the patient-ventilator asynchrony occurs, to a total number of respiratory circulation cycle(s) in the preset time period before the patient-ventilator asynchrony occurs, so as to obtain a frequency for the occurrence of the patient-ventilator asynchrony during the preset time period;
wherein for example, if the preset time period contains ten respiratory circulation cycles, and the patient-ventilator asynchrony occurs within six respiratory circulation cycles, the frequency for the occurrence of the patient-ventilator asynchrony is 60%
step 3220, displaying, on the display interface, a second mark 2, which qualitatively and/or quantitatively characterizes the frequency for the occurrence.

The second mark 2 can be at least one of: dynamic trend graph, broken line graph, scatter graph, spider web graph, number, text, scale graph and dial graph, or can also be other ways that can qualitatively or quantitatively characterize the frequency for the occurrence of the patient-ventilator asynchrony. As shown in FIG. 8, it is a quantitative display of the frequency for the occurrence of patient-ventilator asynchrony. In this figure, the frequency for the occurrence of patient-ventilator asynchrony is directly displayed in a digital form, which is clear to user at a glance.

The frequency for the occurrence of the patient-ventilator asynchrony mentioned above is calculated by integrating different types of the patient-ventilator asynchrony, and can also be referred to as the total frequency for the occurrence of patient-ventilator asynchrony. For different types of patient-ventilator asynchrony, the frequency for the occurrence of each type of patient-ventilator asynchrony can also be calculated separately. Specifically, a second correspondence relationship can be pre-stored, which represents different second marks 2 which correspond to each type of patient-ventilator asynchrony. It is easy to understand that different types of patient-ventilator asynchrony correspond to different second marks 2. The types of patient-ventilator asynchrony occurred within a preset time period, are recognized. A proportion of a number of respiratory circulation cycle(s), in which each type of patient-ventilator asynchrony occurs, to a total number of respiratory circulation cycles in a preset time period, is calculated. Then the frequency for the occurrence of each type of patient-ventilator asynchrony during the preset time period, is obtained. For example, the preset time period includes ten respiratory circulation cycles. If the patient-ventilator asynchrony occurs within six respiratory circulation cycles, the total frequency for the occurrence of patient-ventilator asynchrony is 60%. Among the six respiratory circulation cycles in which the patient-ventilator asynchrony occurs, four respiratory circulation cycles only have invalid triggering, and each respiratory circulation cycle in the other two respiratory circulation cycles has both abnormal triggering and double triggering. The frequency for the occurrence of invalid triggering is 40%, and the occurrence frequencies of abnormal triggering and double triggering are both 20%. After obtaining the frequency for the occurrence of different types of the patient-ventilator asynchrony within the preset time period, the second mark 2, which corresponds to the type of the patient-ventilator asynchrony recognized according to the second correspondence relationship, is displayed. As shown in FIG. 8, in an embodiment, the total frequency for the occurrence of the patient-ventilator asynchrony and the occurrence frequencies of different types of the patient-ventilator asynchrony are displayed on the same display interface. It can be seen that the total frequency for the occurrence of patient-ventilator asynchrony is 15.6%. In FIG. 8, the second mark 2 for each type of patient-ventilator asynchrony includes corresponding characters, where the characters of the second mark 2 corresponding to the invalid triggering are IE, and the characters of the second mark 2 corresponding to the double triggering are DT. The second marks 2 for other types of patient-ventilator asynchrony have the same principles.

Through the above methods, the user can clearly know the total frequency for the occurrence of patient-ventilator asynchrony and the occurrence frequencies of different types of the patient-ventilator asynchrony.

In some embodiments, after step 3220, the method further includes:
Step 3221a, displaying a patient-ventilator asynchrony index. The patient-ventilator asynchrony index is a sum of the occurrence frequencies of different types of the patient-ventilator asynchrony obtained above. The patient-ventilator asynchrony index is configured to characterize a degree of severity of the patient-ventilator asynchrony. Obviously, the higher the patient-ventilator asynchrony index, the relatively higher the degree of severity of the patient-ventilator asynchrony.

In order to make the reminder of the degree of severity of the patient-ventilator asynchrony more obvious, in some embodiments, a third mark 3 is also displayed on the display interface, which includes but is not limited to text, number, symbol, and animation. In some embodiments, whether the patient-ventilator asynchrony index exceeds a preset threshold, is determined. If yes, a characteristic of the third marker 3 can be changed. The characteristic of the third marker 3 can include but is not limited to a color characteristic, brightness characteristic, and pattern characteristic. For example, as shown in FIGS. 9 and 10, the third marker 3 includes a rectangular box and a pointer. An upper half of the rectangular box is of one color (such as red), and a lower half of the rectangular box is of another color (or can also be transparent). Depending on the patient-ventilator asynchrony index, the pointer stays at different heights of the rectangular box. As shown in FIG. 9, when the patient-ventilator asynchrony index does not reach the preset threshold, The display position of the pointer is in the lower half of the rectangular box, as shown in FIG. 10. When the patient-ventilator asynchrony index exceeds the preset threshold, the display position of the pointer is in the upper half of the rectangular box. In this way, the user does not need to see the frequency for the occurrence of patient-ventilator asynchrony or the specific figure of the patient-ventilator asynchrony index, but can more intuitively obtain the degree of severity of patient-ventilator asynchrony through the characteristic change of the third marker 3.

In some embodiments, after step 3220, the method further includes:
Step 3221b, ranking different types of the patient-ventilator asynchrony according to their occurrence frequencies, and displaying a ranking result on the display interface;
wherein as shown in FIG. 11, the frequency for the occurrence of each type of patient-ventilator asynchrony decreases from top to bottom, and the user can preferentially take measures to reduce the types of patient-ventilator asynchrony occurred most frequently;
step 3222, displaying an adjustment suggestion for the ventilation parameter according to a third correspondence relationship. The third correspondence relationship characterizes different adjustment suggestions for ventilation parameter corresponding to different ranking results.

Step 3222 enables the user, who do not need to have too deep medical knowledge, to use the above-mentioned ventilation device well. The usability of the ventilation device in this embodiment is improved. The ventilation parameters recommended can be displayed in the form of text, animation, or image. For example, in FIG. 11, the text "Too much patient-ventilator asynchrony, please reduce the sensitivity of the inhalation trigger" is used as the ventilation parameters recommended. Furthermore, the corresponding relationships between various ranking results and adjustment suggestions for ventilation parameters can include but are not limited to the following. (1) Provide adjustment suggestions for ventilation parameters according to the type of patient-ventilator asynchrony ranked first. (2) Provide adjustment suggestions for ventilation parameters according to the types of patient-ventilator asynchrony, ranked second and third, based on a certain type of patient-ventilator asynchrony ranked first. (3) Provide a corresponding adjustment suggestion for ventilation parameters for each possible ranking result. In other embodiments, a pre-generated ventilation parameter adjustment interface can also be displayed according to an input instruction, which can instruct the user to make parameter adjustments. In some embodiments, the third correspondence relationship can also be prepared according to the physiological parameters of the patient, such as blood oxygen, pulse, and respiratory rate, to provide relevant recommendation for ventilation parameters.

The indication information in the above embodiments can be displayed separately. In some embodiments, the indication information in the above embodiments can also be combined to provide the user with comprehensive indication information about patient-ventilator asynchrony, as shown in FIGS. 12 to 15. As shown in FIG. 12, the left area is frozen waveforms of parameters, while the right area is configured to display occurrence frequencies of different types of the patient-ventilator asynchrony and total frequency for the occurrence of patient-ventilator asynchrony. The first mark 1 of a triangle is displayed on a coordinate axis of a parameter waveform in the figure. In this interface, the total frequency for the occurrence of patient-ventilator asynchrony calculated is 15.6%, which reaches the degree of severity, and an alarm is required in this embodiment. Therefore, alarm information "!!! Excessive patient-ventilator asynchrony" is displayed at a top of the display interface. That is to say, FIG. 12 shows the time point for the occurrence, frequency for the occurrence, and related alarm information of patient-ventilator asynchrony. According to this, the display interface in FIG. 12 also provides the user with corresponding jump or pop-up buttons. For example, the user can click on the area where the total frequency for the occurrence of patient-ventilator asynchrony occurs on the display interface, and a tool for analyzing patient-ventilator asynchrony pops up, as shown in FIG. 15. In this display interface, the tool for analyzing patient-ventilator asynchrony is displayed, and adjustment suggestions for ventilation parameters are displayed. For example, the user can click on the area where the alarm information is located on the display interface to jump to a detailed alarm information interface shown in FIG. 13, which displays more detailed alarm information. If the user further clicks on the alarm information in the detailed alarm information interface, it jumps to the display interface shown in FIG. 14, which displays general adjustment suggestions for ventilation parameters, and the display interface also provides an entry point for the tool for analyzing patient-ventilator asynchrony. After clicking on this button, the tool for analyzing patient-ventilator asynchrony as shown in FIG. 15 pops up.

In the above implementation example, if the patient-ventilator asynchrony occurs, indication information related to patient-ventilator asynchrony and parameter waveforms generated according to the parameter are output. Through the indication information, at least the occurrence time, frequency, degree of severity of the patient-ventilator asynchrony, as well as analysis and comparison information of the patient-ventilator asynchrony can be presented, providing reference for the user to develop ventilation strategies.

The foregoing specific description has been described with reference to various embodiments. However, those skilled in the art can recognize that these various embodiments are only for assisting in understanding rather than limitation. For those skilled in the art, several simple deductions, deformations, or substitutions can also be made according to the ideas of the present disclosure.

The scope of the present invention is defined by the claims that follow.

## Claims

1. A ventilation device, comprising:
a gas source interface (10), which is connected with an external gas source;
a patient interface (40), which is connected with a respiratory system of a patient;
a respiratory line (30), which is connected with both the gas source interface (10) and the patient interface (40), for inputting a gas from the external gas source to the patient and receiving a gas exhaled by the patient;
a drive apparatus (20), which is configured for providing respiratory support power, so as to control to input the gas from the external gas source to the patient;
a **memory** (60);
and
a processor (70), which is configured for acquiring a parameter of the patient which characterizes occurrence of patient-ventilator asynchrony during a ventilation process, wherein the parameter comprises at least one of: a ventilation parameter of the ventilation device and a physiological parameter of the patient;
wherein the processor (70) is further configured for determining whether the patient-ventilator asynchrony occurs according to the parameter, and outputting, when the patient-ventilator asynchrony occurs, indication information, which is associated with the patient-ventilator asynchrony, and parameter waveforms, which are generated according to the parameter;
wherein outputting indication information, which is associated with the patient-ventilator asynchrony, and parameter waveforms, which are generated according to the parameter, comprises: displaying, on a display interface, the indication information, which is associated with the patient-ventilator asynchrony, and the parameter waveforms of a period of time; wherein the patient-ventilator asynchrony occurs within said period of time;
wherein displaying, on a display interface, the indication information, which is associated with the patient-ventilator asynchrony, and the parameter waveforms of a period of time, comprises: recording, through the memory (60), a time point when the patient-ventilator asynchrony occurs; and
displaying a first mark (1) at a position of the parameter waveforms, which position corresponds to the time point when the patient-ventilator asynchrony occurs;
**characterized in that**,
displaying a first mark (1) at a position of the parameter waveforms, which position corresponds to the time point when the patient-ventilator asynchrony occurs, comprises:
recognizing, through the processor (70), a type of the patient-ventilator asynchrony occurred; and
displaying, on the display interface, the first mark (1), which corresponds to the type of the patient-ventilator asynchrony occurred, which type is recognized through the processor (70) according to a first correspondence relationship, wherein the first correspondence relationship characterizes a first mark (1) which corresponds to different types of patient-ventilator asynchrony, wherein the first correspondence relationship is pre-stored in the memory (60);
wherein said first mark (1), which corresponds to different types of patient-ventilator asynchrony, differs in at least one characteristic of color, pattern, and brightness.

2. The ventilation device according to claim 1, **characterized in that**, the indication information comprises at least one of: a time point for the occurrence of the patient-ventilator asynchrony, a frequency for the occurrence of the patient-ventilator asynchrony, information for a degree of the patient-ventilator asynchrony and information for analysis of the patient-ventilator asynchrony; and/or
the parameter comprises at least one of: flow rate, pressure, volume, esophageal pressure, intragastric pressure, transpulmonary pressure, blood oxygen, respiratory rate, transdiaphragmatic pressure, and diaphragmatic electromyography.

3. The ventilation device according to claim 1, **characterized in that**, the first mark (1) comprises at least one of: text, number, symbol, and animation.

4. The ventilation device according to claim 1, **characterized in that**,
displaying, on a display interface, the indication information, which is associated with the patient-ventilator asynchrony, and the parameter waveforms of a period of time, further comprises:
calculating, through the processor (70), a proportion of a number of respiratory circulation cycle(s) in a preset time period, in which cycle(s) the patient-ventilator asynchrony occurs, to a total number of respiratory circulation cycle(s) in the preset time period, so as to obtain a frequency for the occurrence of the patient-ventilator asynchrony during the preset time period; and
displaying, on the display interface, a second mark (2) which characterizes the occurrence frequency, wherein the second mark (2) is pre-stored in the memory (60).

5. The ventilation device according to claim 4, **characterized in that**, the second mark (2) comprises at least one of: dynamic trend graph, broken line graph, scatter graph, spider web graph, number, text, scale graph and dial graph.

6. The ventilation device according to claim 4, **characterized in that**, displaying, on the display interface, a second mark (2), comprises:
recognizing, through the processor (70), types of the patient-ventilator asynchrony occurred in the preset time period;
respectively calculating, through the processor (70), a proportion of the number of respiratory circulation cycle(s) in the preset time period, in which cycle(s) one or more types of patient-ventilator asynchrony occur, to the total number of respiratory circulation cycle(s) in the preset time period, so as to obtain the frequency for the occurrence of each type of the patient-ventilator asynchrony occurred during the preset time period; and
displaying the second mark (2), which corresponds to the type of the patient-ventilator asynchrony occurred, which type is recognized according to a second correspondence relationship, wherein the second correspondence relationship characterizes different second marks which respectively correspond to different types of patient-ventilator asynchrony, wherein the second correspondence relationship is pre-stored in the memory (60).

7. The ventilation device according to claim 1, **characterized in that**,
displaying, on a display interface, the indication information, which is associated with the patient-ventilator asynchrony, and the parameter waveforms of a period of time, further comprises:
respectively calculating, through the processor (70), a proportion of a number of respiratory circulation cycle(s) in the preset time period, in which cycle(s) one or more types of patient-ventilator asynchrony occur, to a total number of respiratory circulation cycle(s) in the preset time period, so as to obtain a frequency for the occurrence of each type of the patient-ventilator asynchrony occurred during the preset time period; and
displaying, on the display interface, a patient-ventilator asynchrony index, which is a sum of the frequencies obtained for the occurrence of each type of the patient-ventilator asynchrony occurred and characterizes a degree of the patient-ventilator asynchrony occurred.

8. The ventilation device according to claim 7, **characterized in that**, the ventilation device is further configured for:
displaying, on the display interface, a third mark (3), which characterizes whether the patient-ventilator asynchrony index exceeds a preset threshold;
wherein the processor (70) is further configured for determining whether the patient-ventilator asynchrony index exceeds the preset threshold, and changing a characteristic of the third mark (3) when the patient-ventilator asynchrony index is determined to exceed the preset threshold, wherein the third mark is pre-stored in the memory (60).

9. The ventilation device according to claim 8, **characterized in that**, the characteristic of the third mark (3) comprises at least one of: color, brightness, and pattern; and/or
the third mark (3) comprises at least one of: text, number, symbol, and animation.

10. The ventilation device according to claim 1, **characterized in that**,
displaying, on a display interface, the indication information, which is associated with the patient-ventilator asynchrony, and the parameter waveforms of a period of time, comprises:
respectively calculating, through the processor (70), a proportion of a number of respiratory circulation cycle(s) in the preset time period, in which cycle(s) one or more types of patient-ventilator asynchrony occur, to a total number of respiratory circulation cycle(s) in the preset time period, so as to obtain a frequency for the occurrence of each type of the patient-ventilator asynchrony occurred during the preset time period;
ranking, through the processor (70), each type of patient-ventilator asynchrony according to the frequency for the occurrence of each type of patient-ventilator asynchrony occurred, to obtain a ranking result; and
displaying an adjustment suggestion for the ventilation parameter corresponding to a type of the patient-ventilator asynchrony ranked first according to a third correspondence relationship, wherein the third correspondence relationship characterizes different adjustment suggestions for the ventilation parameter, which suggestions correspond to different ranking results, wherein the third correspondence relationship is pre-stored in the memory (60);
preferably, further comprising displaying the ranking result on the display interface;
preferably, displaying an adjustment suggestion for the ventilation parameter, comprises:
displaying, through at least one of text, animation, and image, the adjustment suggestion for the ventilation parameter; or
displaying, based on an input instruction, an adjustment interface for the ventilation parameter, which interface is pre-generated by the processor (70).

11. A display method for a ventilation device, comprising:
acquiring a parameter of a patient which characterizes occurrence of patient-ventilator asynchrony during a ventilation process, wherein the parameter comprises at least one of: a ventilation parameter of the ventilation device and a physiological parameter of the patient;
determining whether the patient-ventilator asynchrony occurs according to the parameter; and
outputting, when the patient-ventilator asynchrony occurs, indication information, which is associated with the patient-ventilator asynchrony, and parameter waveforms, which are generated according to the parameter;
wherein outputting indication information, which is associated with the patient-ventilator asynchrony, and parameter waveforms, which are generated according to the parameter, comprises:
displaying, on a display interface, the indication information, which is associated with the patient-ventilator asynchrony, and the parameter waveforms of a period of time;
wherein the patient-ventilator asynchrony occurs within said period of time;
wherein displaying, on a display interface, the indication information, which is associated with the patient-ventilator asynchrony, and the parameter waveforms of a period of time, comprises:
recording a time point when the patient-ventilator asynchrony occurs; and
displaying a first mark (1) at a position of the parameter waveforms, which position corresponds to the time point when the patient-ventilator asynchrony occurs;
**characterized in that**
displaying a first mark (1) at a position of the parameter waveforms, which position corresponds to the time point when the patient-ventilator asynchrony occurs, comprises:
recognizing a type of the patient-ventilator asynchrony occurred; and
displaying the first mark (1), which corresponds to the type of the patient-ventilator asynchrony occurred, which type is recognized according to a first correspondence relationship, wherein the first correspondence relationship characterizes a first mark which corresponds to different types of patient-ventilator asynchrony; wherein said first mark (1), which corresponds to different types of patient-ventilator asynchrony, differs in at least one characteristic of color, pattern, and brightness.

## Patentansprüche

1. Eine Beatmungsvorrichtung, bestehend aus:
einer Gasquellenschnittstelle (10), die mit einer externen Gasquelle verbunden ist;
einer Patientenschnittstelle (40), die mit dem Atmungssystem eines Patienten verbunden ist;
einer Atemleitung (30), die sowohl mit der Gasquellenschnittstelle (10) als auch der Patientenschnittstelle (40) verbunden ist, um dem Patienten Gas aus der externen Gasquelle zuzuführen und vom Patienten ausgeatmetes Gas aufzunehmen;
einer Antriebsvorrichtung (20), die so ausgelegt ist, dass sie eine Atemunterstützung bereitstellt, um die Zufuhr des Gases von der externen Gasquelle zum Patienten zu steuern;
einem Speicher (60);
und
einem Prozessor (70), der zum Erfassen eines Parameters des Patienten ausgelegt ist, der das Auftreten einer Asynchronie zwischen Patienten und Beatmungsgerät während eines Beatmungsvorgangs charakterisiert, wobei der Parameter mindestens einen der folgenden Parameter umfasst: einen Beatmungsparameter des Beatmungsgeräts oder einen physiologischen Parameter des Patienten;
wobei der Prozessor (70) ferner so ausgelegt ist, dass er anhand des Parameters bestimmt, ob die Asynchronie zwischen Patienten und Beatmungsgerät auftritt, und bei Auftreten der Asynchronie zwischen Patienten und Beatmungsgerät Anzeigeinformationen, die mit der Asynchronie zwischen Patienten und Beatmungsgerät in Verbindung stehen, und Parameterwellenformen ausgibt, die gemäß dem Parameter erzeugt werden;
wobei die Ausgabe von Anzeigeinformationen, die mit der Asynchronie zwischen Patienten und Beatmungsgerät in Zusammenhang stehen, und von Parameterwellenformen, die gemäß dem Parameter erzeugt werden, Folgendes umfasst: die Anzeige auf der Anzeigeoberfläche der Anzeigeinformationen, die mit der Asynchronie zwischen Patienten und Beatmungsgerät in Zusammenhang stehen, und der Parameterwellenformen eines Zeitraums, wobei die Asynchronie zwischen Patienten und Beatmungsgerät in besagtem Zeitraum auftritt;
wobei die Anzeige auf einer Anzeigeoberfläche der Anzeigeinformationen, die mit der Asynchronie zwischen Patienten und Beatmungsgerät in Zusammenhang stehen, und der Parameterwellenformen eines Zeitraums Folgendes umfasst: über den Speicher (60) das Aufzeichnen eines Zeitpunkts, zu dem die Asynchronie zwischen Patienten und Beatmungsgerät auftritt; und
das Anzeigen einer ersten Markierung (1) an einer Position der Parameterwellenformen, wobei diese Position dem Zeitpunkt entspricht, zu dem die Asynchronie zwischen Patienten und Beatmungsgerät auftritt;
**dadurch gekennzeichnet, dass**
das Anzeigen einer ersten Markierung (1) an einer Position der Parameterwellenformen, die dem Zeitpunkt entspricht, zu dem die Asynchronie zwischen Patienten und Beatmungsgerät auftritt, Folgendes umfasst:
das Erkennen einer Art der aufgetretenen Asynchronie zwischen Patienten und Beatmungsgerät durch den Prozessor (70); und
das Anzeigen auf der Anzeigeoberfläche der ersten Markierung (1), die der Art der aufgetretenen Asynchronie zwischen Patienten und Beatmungsgerät entspricht und deren Typ durch den Prozessor (70) gemäß einer ersten Entsprechungsbeziehung erkannt wird, wobei die erste Entsprechungsbeziehung eine erste Markierung (1) charakterisiert, die verschiedenen Arten von Asynchronien zwischen Patienten und Beatmungsgerät entspricht, wobei die erste Entsprechungsbeziehung im Speicher (60) vorab gespeichert wird;
wobei
sich die besagte erste Markierung (1), die verschiedenen Arten von Asynchronien zwischen Patienten und Beatmungsgerät entspricht, in mindestens einem Merkmal (Farbe, Muster oder Helligkeit) unterscheidet.

2. Die Beatmungsvorrichtung gemäß Anspruch 1, **dadurch gekennzeichnet, dass** die Anzeigeinformationen mindestens eines der folgenden Elemente umfassen: einen Zeitpunkt für das Auftreten der der Asynchronie zwischen Patienten und Beatmungsgerät, eine Häufigkeit für das Auftreten der Asynchronie zwischen Patienten und Beatmungsgerät, Informationen über den Grad der Asynchronie zwischen Patienten und Beatmungsgerät und Informationen zur Analyse der Asynchronie zwischen Patienten und Beatmungsgerät; und/oder
der Parameter mindestens eines der folgenden Elemente umfasst: Flussrate, Druck, Volumen, Ösophagusdruck, intragastrischer Druck, transpulmonaler Druck, Blutsauerstoff, Atemfrequenz, transdiaphragmatischer Druck und diaphragmatische Elektromyographie.

3. Die Beatmungsvorrichtung gemäß Anspruch 1, **dadurch gekennzeichnet, dass** die erste Markierung (1) mindestens eines der folgenden Elemente umfasst: Text, Zahl, Symbol oder Animation.

4. Die Beatmungsvorrichtung gemäß Anspruch 1, **dadurch gekennzeichnet, dass** sie auf einer Anzeigeoberfläche die Anzeigeinformationen, die mit der Asynchronie zwischen Patienten und Beatmungsgerät verbunden sind, und die Parameterwellenformen eines Zeitraums anzeigt und ferner Folgendes umfasst:
das Berechnen durch den Prozessor (70) eines Anteils einer Anzahl von Atemzyklen in einem voreingestellten Zeitabschnitt, in denen die Asynchronie zwischen Patienten und Beatmungsgerät auftritt, in Bezug auf die Gesamtzahl von Atemzyklen in dem voreingestellten Zeitabschnitt, um eine Häufigkeit für das Auftreten der Asynchronie zwischen Patienten und Beatmungsgerät während des voreingestellten Zeitabschnitts zu ermitteln; und
das Anzeigen einer zweiten Markierung (2) auf der Anzeigeoberfläche, die die Auftrittshäufigkeit charakterisiert, wobei die zweite Markierung (2) vorab im Speicher (60) hinterlegt wird.

5. Die Beatmungsvorrichtung gemäß Anspruch 4, **dadurch gekennzeichnet, dass** die zweite Markierung (2) mindestens eines der folgenden Elemente umfasst: dynamisches Trenddiagramm, gestricheltes Liniendiagramm, Streudiagramm, Spinnennetzdiagramm, Zahl, Text, Skalendiagramm oder Zifferblattdiagramm.

6. Die Beatmungsvorrichtung gemäß Anspruch 4, **dadurch gekennzeichnet, dass** die Anzeige einer zweiten Markierung (2) auf der Anzeigeoberfläche Folgendes umfasst:
das Erkennen von Arten der zwischen Patienten und Beatmungsgerät aufgetretenen Asynchronie in dem voreingestellten Zeitraum durch den Prozessor (70);
die jeweilige Berechnung durch den Prozessor (70) eines Anteils der Anzahl der Atemzyklen in dem voreingestellten Zeitraum, in denen eine oder mehrere Arten von Asynchronie zwischen Patienten und Beatmungsgerät auftreten, in Bezug auf die Gesamtzahl der Atemzyklen in dem voreingestellten Zeitraum, um die Häufigkeit des Auftretens jeder Art von Asynchronie zwischen Patienten und Beatmungsgerät während des voreingestellten Zeitraums zu ermitteln; und
das Anzeigen der zweiten Markierung (2), die der Art der aufgetretenen Asynchronie zwischen Patienten und Beatmungsgerät entspricht, wobei diese Art gemäß einer zweiten Zuordnungsbeziehung erkannt wird,
wobei die zweite Zuordnungsbeziehung unterschiedliche zweite Markierungen charakterisiert, die jeweils unterschiedlichen Arten von Asynchronie zwischen Patienten und Beatmungsgerät entsprechen, wobei die zweite Zuordnungsbeziehung im Speicher (60) vorab hinterlegt ist.

7. Die Beatmungsvorrichtung gemäß Anspruch 1, **dadurch gekennzeichnet, dass** das Anzeigen der Anzeigeinformationen, die mit der Asynchronie zwischen Patienten und Beatmungsgerät verbunden sind, und der Parameterwellenformen eines Zeitraums auf einer Anzeigeoberfläche, ferner Folgendes umfasst:
die jeweilige Berechnung durch den Prozessor (70) eines Anteils einer Anzahl von Atemzyklen in dem voreingestellten Zeitintervall, in denen eine oder mehrere Arten von Asynchronie zwischen Patienten und Beatmungsgerät auftreten, in Bezug zu einer Gesamtzahl von Atemzyklen in dem voreingestellten Zeitraum, um eine Häufigkeit für das Auftreten jeder Art der Asynchronie zwischen Patienten und Beatmungsgerät zu ermitteln, die während des voreingestellten Zeitraums aufgetreten ist; und
das Anzeigen einer Kennzahl für die Asynchronie zwischen Patienten und Beatmungsgerät auf der Anzeigeoberfläche, die einer Summe der Frequenzen entspricht, die für das Auftreten jeder Art der aufgetretenen Asynchronie zwischen Patienten und Beatmungsgerät ermittelt wurden, und die einen Grad der aufgetretenen Asynchronie zwischen Patienten und Beatmungsgerät charakterisiert.

8. Die Beatmungsvorrichtung gemäß Anspruch 7, **dadurch gekennzeichnet, dass** das Beatmungsgerät ferner für Folgendes ausgelegt ist:
das Anzeigen einer dritten Markierung (3) auf der Anzeigeoberfläche, die charakterisiert, ob die Kennzahl für die Asynchronie zwischen Patienten und Beatmungsgerät einen voreingestellten Schwellenwert überschreitet;
wobei der Prozessor (70) ferner dafür ausgelegt ist, zu bestimmen, ob die Kennzahl für die Asynchronie zwischen Patienten und Beatmungsgerät den voreingestellten Schwellenwert überschreitet, und eine Eigenschaft der dritten Markierung (3) zu ändern, wenn festgestellt wird, dass die Kennzahl für die Asynchronie zwischen Patienten und Beatmungsgerät den voreingestellten Schwellenwert überschreitet, wobei die dritte Markierung im Speicher (60) vorab hinterlegt ist.

9. Die Beatmungsvorrichtung gemäß Anspruch 8, **dadurch gekennzeichnet, dass** die Eigenschaft der dritten Markierung (3) mindestens eines der folgenden Merkmale umfasst: Farbe, Helligkeit und Muster; und/oder
die dritte Markierung (3) mindestens eines der folgenden Elemente umfasst: Text, Zahl, Symbol oder Animation.

10. . Die Beatmungsvorrichtung gemäß Anspruch 1, **dadurch gekennzeichnet, dass** das Anzeigen der Anzeigeinformationen, die mit der Asynchronie zwischen Patienten und Beatmungsgerät verbunden sind, und der Parameterwellenformen eines Zeitraums auf einer Anzeigeoberfläche Folgendes umfasst:
die jeweilige Berechnung durch den Prozessor (70) eines Anteils einer Anzahl von Atemzyklen in dem voreingestellten Zeitabschnitt, in denen eine oder mehrere Arten von Asynchronie zwischen Patienten und Beatmungsgerät auftreten, in Bezug auf eine Gesamtzahl von Atemzyklen in dem voreingestellten Zeitabschnitt, um eine Häufigkeit für das Auftreten jeder Art der Asynchronie zwischen Patienten und Beatmungsgerät zu ermitteln, die während des vorgegebenen Zeitraums aufgetreten ist;
das Ordnen durch den Prozessor (70) jeder Art von Asynchronie zwischen Patienten und Beatmungsgerät entsprechend der Häufigkeit des Auftretens der jeweiligen Art von Asynchronie zwischen Patienten und Beatmungsgerät, um ein Rangordnungsergebnis zu erhalten; und
das Anzeigen eines Anpassungsvorschlags für die Beatmungsparameter entsprechend einem Typ der Asynchronie zwischen Patienten und Beatmungsgerät, der gemäß einer dritten Entsprechungsbeziehung an erster Stelle steht, wobei die dritte Entsprechungsbeziehung verschiedene Einstellungsvorschläge für den Beatmungsparameter charakterisiert und diese Vorschläge verschiedenen Rangordnungsergebnissen entsprechen, wobei die dritte Entsprechungsbeziehung im Speicher (60) vorab hinterlegt ist;
vorzugsweise ferner umfassend das Anzeigen des Rangordnungsergebnisses auf der Anzeigeoberfläche;
vorzugsweise umfasst das Anzeigen eines Einstellungsvorschlags für den Beatmungsparameter:
das Anzeigen des Anpassungsvorschlags für den Beatmungsparameter durch mindestens einen Text, eine Animation oder ein Bild; oder
das Anzeigen einer Anpassungsoberfläche für den Beatmungsparameter auf der Grundlage einer Eingabeanweisung, wobei die Oberfläche vom Prozessor (70) vorab generiert wird.

11. Ein Anzeigeverfahren für eine Beatmungsvorrichtung, das Folgendes umfasst:
das Erfassen eines Parameters eines Patienten, der das Auftreten einer Asynchronie zwischen Patienten und Beatmungsgerät während eines Beatmungsvorgangs charakterisiert, wobei der Parameter mindestens einen der folgenden Parameter umfasst: einen Beatmungsparameter des Beatmungsgeräts oder einen physiologischen Parameter des Patienten;
das Bestimmen, ob die Asynchronie zwischen Patienten und Beatmungsgerät gemäß dem Parameter auftritt; und
wenn die Asynchronie zwischen Patienten und Beatmungsgerät auftritt, die Ausgabe von Anzeigeinformationen, die mit der Asynchronie zwischen Patienten und Beatmungsgerät in Zusammenhang stehen, und von Parameterwellenformen, die gemäß dem Parameter erzeugt werden;
wobei das Ausgeben von Anzeigeinformationen, die mit der Asynchronie zwischen Patienten und Beatmungsgerät in Zusammenhang stehen, und von Parameterwellenformen, die gemäß dem Parameter erzeugt werden, Folgendes umfasst:
das Anzeigen der Anzeigeinformationen, die mit der Asynchronie zwischen Patienten und Beatmungsgerät in Zusammenhang stehen, und der Parameterwellenformen eines Zeitraums auf einer Anzeigeoberfläche;
wobei die Asynchronie zwischen Patienten und Beatmungsgerät innerhalb des besagten Zeitraums auftritt;
wobei die Anzeige von Anzeigeinformationen, die mit der Asynchronie zwischen Patienten und Beatmungsgerät verbunden sind, und die Parameterwellenformen eines Zeitraums auf einer Anzeigeoberfläche Folgendes umfasst:
das Aufzeichnen eines Zeitpunkts, zu dem die Asynchronie zwischen Patienten und Beatmungsgerät auftritt; und
das Anzeigen einer ersten Markierung (1) an einer Position der Parameterwellenformen, wobei diese Position dem Zeitpunkt entspricht, zu dem die Asynchronie zwischen Patienten und Beatmungsgerät auftritt;
**dadurch gekennzeichnet, dass**
das Anzeigen einer ersten Markierung (1) an einer Position der Parameterwellenformen, die dem Zeitpunkt entspricht, zu dem die Asynchronie zwischen Patienten und Beatmungsgerät auftritt, Folgendes umfasst:
das Erkennen einer Art der aufgetretenen Asynchronie zwischen Patienten und Beatmungsgerät; und
das Anzeigen der ersten Markierung (1), die der Art der aufgetretenen Asynchronie zwischen Patienten und Beatmungsgerät entspricht, wobei die erste Entsprechungsbeziehung eine erste Markierung charakterisiert, die verschiedenen Arten von Asynchronie zwischen Patienten und Beatmungsgerät entspricht; wobei
sich die besagte erste Markierung (1), die verschiedenen Arten der Asynchronie zwischen Patienten und Beatmungsgerät entspricht, in mindestens einem Merkmal (Farbe, Muster oder Helligkeit) unterscheidet.

## Revendications

1. Dispositif de ventilation, comprenant :
une interface de source de gaz (10), laquelle est reliée à une source de gaz externe ;
une interface patient (40), laquelle est reliée à un système respiratoire d'un patient ;
une ligne respiratoire (30), laquelle est reliée à la fois à l'interface de source de gaz (10) et à l'interface patient (40), destinée à délivrer un gaz provenant de la source de gaz externe au patient et à recevoir un gaz exhalé par le patient ;
un appareil d'entraînement (20), lequel est configuré pour fournir une puissance d'assistance respiratoire ; de sorte à contrôler la délivrance du gaz provenant de la source de gaz externe au patient ;
une mémoire (60) ;
et
un processeur (70), lequel est configuré pour acquérir un paramètre du patient lequel caractérise la survenue d'une asynchronie entre le patient et le ventilateur durant un processus de ventilation, où le paramètre comprend au moins l'un : d'un paramètre de ventilation du dispositif de ventilation et d'un paramètre physiologique du patient ;
où le processeur (70) est en outre configuré pour déterminer si l'asynchronie entre le patient et le ventilateur survient en fonction du paramètre, et délivrer, lorsque l'asynchronie entre le patient et le ventilateur survient, une information d'indication, laquelle est associée à l'asynchronie entre le patient et le ventilateur, et des formes d'onde de paramètre, lesquelles sont générées en fonction du paramètre ;
où le fait de délivrer une information d'indication, laquelle est associée à l'asynchronie entre le patient et le ventilateur, et des formes d'onde de paramètre, lesquelles sont générées en fonction du paramètre, comprend : le fait d'afficher, sur une interface d'affichage, l'information d'indication, laquelle est associée à l'asynchronie entre le patient et le ventilateur, et les formes d'onde de paramètre d'un intervalle de temps ; où l'asynchronie entre le patient et le ventilateur survient au cours dudit intervalle de temps ;
où le fait d'afficher, sur une interface d'affichage, l'information d'indication, laquelle est associée à l'asynchronie entre le patient et le ventilateur, et les formes d'onde de paramètre d'un intervalle de temps, comprend : le fait d'enregistrer, via la mémoire (60), un instant auquel l'asynchronie entre le patient et le ventilateur survient ; et le fait d'afficher une première marque (1) à une position des formes d'onde de paramètre, laquelle position correspond à l'instant auquel l'asynchronie entre le patient et le ventilateur survient ;
**caractérisé en ce que**,
le fait d'afficher une première marque (1) à une position des formes d'onde de paramètre, laquelle position correspond à l'instant auquel l'asynchronie entre le patient et le ventilateur survient, comprend :
le fait de reconnaître, via le processeur (70), un type de l'asynchronie entre le patient et le ventilateur ayant eu lieu ; et
le fait d'afficher, sur l'interface d'affichage, la première marque (1), laquelle correspond au type de l'asynchronie entre le patient et le ventilateur ayant eu lieu, lequel type est reconnu via le processeur (70) en fonction d'une première relation de correspondance, où la première relation de correspondance caractérise une première marque (1) laquelle correspond à différents types d'asynchronie entre le patient et le ventilateur, où la première relation de correspondance est pré-mémorisée dans la mémoire (60) ;
où ladite première marque (1), laquelle correspond à différents types d'asynchronie entre le patient et le ventilateur, diffère par au moins une caractéristique de couleur, de motif, et de luminosité.

2. Dispositif de ventilation selon la revendication 1, **caractérisé en ce que**, l'information d'indication comprend au moins l'un : d'un instant de la survenue de l'asynchronie entre le patient et le ventilateur, d'une fréquence de la survenue de l'asynchronie entre le patient et le ventilateur, d'une information concernant un degré de l'asynchronie entre le patient et le ventilateur et d'une information concernant l'analyse de l'asynchronie entre le patient et le ventilateur ; et/ou
le paramètre comprend au moins l'un : d'un débit, d'une pression, d'un volume, d'une pression œsophagienne, d'une pression intragastrique, d'une pression transpulmonaire, d'une oxygénation du sang, d'une fréquence respiratoire, d'une pression transdiaphragmatique, et d'une électromyographie diaphragmatique.

3. Dispositif de ventilation selon la revendication 1, **caractérisé en ce que**, la première marque (1) comprend au moins l'un : d'un texte, d'un chiffre, d'un symbole, et d'une animation.

4. Dispositif de ventilation selon la revendication 1, **caractérisé en ce que**,
le fait d'afficher, sur une interface d'affichage, l'information d'indication, laquelle est associée à l'asynchronie entre le patient et le ventilateur, et les formes d'onde de paramètre d'un intervalle de temps, comprend en outre :
le fait de calculer, via le processeur (70), une proportion d'un nombre de cycle(s) de circulation respiratoire(s) dans un intervalle de temps prédéfini, au cours duquel(desquels) cycle(s) l'asynchronie entre le patient et le ventilateur survient, par rapport à un nombre total de cycle(s) de circulation respiratoire(s) dans l'intervalle de temps prédéfini, de sorte à obtenir une fréquence de la survenue de l'asynchronie entre le patient et le ventilateur durant l'intervalle de temps prédéfini ; et
le fait d'afficher, sur l'interface d'affichage, une deuxième marque (2) laquelle caractérise la fréquence de survenue, où la deuxième marque (2) est pré-mémorisée dans la mémoire (60).

5. Dispositif de ventilation selon la revendication 4, **caractérisé en ce que**, la deuxième marque (2) comprend au moins l'un : d'un graphique de tendance dynamique, d'un graphique de ligne brisée, d'un graphique en nuage de points, d'un graphique en toile d'araignée, d'un chiffre, d'un texte, d'un graphique d'échelle et d'un graphique en jauge.

6. Dispositif de ventilation selon la revendication 4, **caractérisé en ce que**, le fait d'afficher, sur l'interface d'affichage, une deuxième marque (2), comprend :
le fait de reconnaître, via le processeur (70), des types de l'asynchronie entre le patient et le ventilateur ayant eu lieu dans l'intervalle de temps prédéfini ;
le fait de respectivement calculer, via le processeur (70), une proportion du nombre de cycle(s) de circulation respiratoire(s) dans l'intervalle de temps prédéfini, au cours duquel(desquels) cycle(s) un ou plusieurs types d'asynchronie entre le patient et le ventilateur surviennent, par rapport au nombre total de cycle(s) de circulation respiratoire(s) dans l'intervalle de temps prédéfini, de sorte à obtenir la fréquence de la survenue de chaque type de l'asynchronie entre le patient et le ventilateur ayant eu lieu durant l'intervalle de temps prédéfini ; et
le fait d'afficher la deuxième marque (2), laquelle correspond au type de l'asynchronie entre le patient et le ventilateur ayant eu lieu, lequel type est reconnu en fonction d'une deuxième relation de correspondance, où la deuxième relation de correspondance caractérise différentes deuxièmes marques lesquelles correspondent respectivement à différents types d'asynchronie entre le patient et le ventilateur, où la deuxième relation de correspondance est pré-mémorisée dans la mémoire (60).

7. Dispositif de ventilation selon la revendication 1, **caractérisé en ce que**,
le fait d'afficher, sur une interface d'affichage, l'information d'indication, laquelle est associée à l'asynchronie entre le patient et le ventilateur, et les formes d'onde de paramètre d'un intervalle de temps, comprend en outre :
le fait de respectivement calculer, via le processeur (70), une proportion d'un nombre de cycle(s) de circulation respiratoire(s) dans l'intervalle de temps prédéfini, au cours duquel(desquels) cycle(s) un ou plusieurs types d'asynchronie entre le patient et le ventilateur surviennent, par rapport à un nombre total de cycle(s) de circulation respiratoire(s) dans l'intervalle de temps prédéfini, de sorte à obtenir une fréquence de la survenue de chaque type de l'asynchronie entre le patient et le ventilateur ayant eu lieu durant l'intervalle de temps prédéfini ; et
le fait d'afficher, sur l'interface d'affichage, un indice d'asynchronie entre le patient et le ventilateur, lequel est la somme des fréquences obtenues de la survenue de chaque type de l'asynchronie entre le patient et le ventilateur ayant eu lieu et caractérise un degré de l'asynchronie entre le patient et le ventilateur ayant eu lieu.

8. Dispositif de ventilation selon la revendication 7, **caractérisé en ce que**, le dispositif de ventilation est en outre configuré pour :
afficher, sur l'interface d'affichage, une troisième marque (3), laquelle caractérise si l'indice d'asynchronie entre le patient et le ventilateur dépasse un seuil prédéfini ;
où le processeur (70) est en outre configuré pour déterminer si l'indice d'asynchronie entre le patient et le ventilateur dépasse le seuil prédéfini, et changer une caractéristique de la troisième marque (3) lorsqu'il est identifié que l'indice d'asynchronie entre le patient et le ventilateur dépasse le seuil prédéfini, où la troisième marque est pré-mémorisée dans la mémoire (60).

9. Dispositif de ventilation selon la revendication 8, **caractérisé en ce que**, la caractéristique de la troisième marque (3) comprend au moins l'un : d'une couleur, d'une luminosité, et d'un motif ; et/ou
la troisième marque (3) comprend au moins l'un : d'un texte, d'un chiffre, d'un symbole, et d'une animation.

10. Dispositif de ventilation selon la revendication 1, **caractérisé en ce que**,
le fait d'afficher, sur une interface d'affichage, l'information d'indication, laquelle est associée à l'asynchronie entre le patient et le ventilateur, et les formes d'onde de paramètre d'un intervalle de temps, comprend :
le fait de respectivement calculer, via le processeur (70), une proportion d'un nombre de cycle(s) de circulation respiratoire(s) dans l'intervalle de temps prédéfini, au cours duquel(desquels) cycle(s) un ou plusieurs types d'asynchronie entre le patient et le ventilateur surviennent, par rapport à un nombre total de cycle(s) de circulation respiratoire(s) dans l'intervalle de temps prédéfini, de sorte à obtenir une fréquence de la survenue de chaque type de l'asynchronie entre le patient et le ventilateur ayant eu lieu durant l'intervalle de temps prédéfini ;
le fait de classer, via le processeur (70), chaque type d'asynchronie entre le patient et le ventilateur en fonction de la fréquence de la survenue de chaque type d'asynchronie entre le patient et le ventilateur ayant eu lieu, pour obtenir un résultat de classement ; et
le fait d'afficher une suggestion de réglage pour le paramètre de ventilation correspondant à un type de l'asynchronie entre le patient et le ventilateur classé en premier en fonction d'une troisième relation de correspondance, où la troisième relation de correspondance caractérise différentes suggestions de réglage pour le paramètre de ventilation, lesquelles suggestions correspondent à différents résultats de classement, où la troisième relation de correspondance est pré-mémorisée dans la mémoire (60) ;
de préférence, comprenant en outre le fait d'afficher le résultat de classement sur l'interface d'affichage ;
de préférence, le fait d'afficher une suggestion de réglage pour le paramètre de ventilation comprend :
le fait d'afficher, via au moins l'un d'un texte, d'une animation, et d'une image, la suggestion de réglage pour le paramètre de ventilation ; ou
le fait d'afficher, sur la base d'une instruction d'entrée, une interface de réglage pour le paramètre de ventilation, laquelle interface est pré-générée par le processeur (70).

11. Procédé d'affichage destiné à un dispositif de ventilation, comprenant les étapes consistant à :
acquérir un paramètre d'un patient lequel caractérise la survenue d'une asynchronie entre le patient et le ventilateur durant un processus de ventilation, où le paramètre comprend au moins l'un : d'un paramètre de ventilation du dispositif de ventilation et d'un paramètre physiologique du patient ;
déterminer si l'asynchronie entre le patient et le ventilateur survient en fonction du paramètre ; et
délivrer, lorsque l'asynchronie entre le patient et le ventilateur survient, une information d'indication, laquelle est associée à l'asynchronie entre le patient et le ventilateur, et des formes d'onde de paramètre, lesquelles sont générées en fonction du paramètre ;
où le fait de délivrer une information d'indication, laquelle est associée à l'asynchronie entre le patient et le ventilateur, et des formes d'onde de paramètre, lesquelles sont générées en fonction du paramètre, comprend :
le fait d'afficher, sur une interface d'affichage, l'information d'indication, laquelle est associée à l'asynchronie entre le patient et le ventilateur, et les formes d'onde de paramètre d'un intervalle de temps ;
où l'asynchronie entre le patient et le ventilateur survient au cours dudit intervalle de temps ;
où le fait d'afficher, sur une interface d'affichage, l'information d'indication, laquelle est associée à l'asynchronie entre le patient et le ventilateur, et les formes d'onde de paramètre d'un intervalle de temps, comprend :
le fait d'enregistrer un instant auquel l'asynchronie entre le patient et le ventilateur survient ; et
le fait d'afficher une première marque (1) à une position des formes d'onde de paramètre, laquelle position correspond à l'instant auquel l'asynchronie entre le patient et le ventilateur survient ;
**caractérisé en ce que**
le fait d'afficher une première marque (1) à une position des formes d'onde de paramètre, laquelle position correspond à l'instant auquel l'asynchronie entre le patient et le ventilateur survient, comprend :
le fait de reconnaître un type de l'asynchronie entre le patient et le ventilateur ayant eu lieu ; et
le fait d'afficher la première marque (1), laquelle correspond au type de l'asynchronie entre le patient et le ventilateur ayant eu lieu, lequel type est reconnu en fonction d'une première relation de correspondance, où la première relation de correspondance caractérise une première marque laquelle correspond à différents types d'asynchronie entre le patient et le ventilateur ; où
ladite première marque (1), laquelle correspond à différents types d'asynchronie entre le patient et le ventilateur, diffère par au moins une caractéristique de couleur, de motif, et de luminosité.
